(19)

Europäisches
Patentamt
European
Patent Office
Office européen
des brevets

(11) **EP 2 942 055 A1**

(12) **EUROPEAN PATENT APPLICATION**

(43) Date of publication:
**11.11.2015 Bulletin 2015/46**

(21) Application number: **15171980.4**

(22) Date of filing: **11.02.2011**

(51) Int Cl.:
*A61K 9/20* (2006.01)  *G01N 33/94* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.02.2010 US 303467 P**

(62) Document number(s) of the earlier application(s) in
accordance with Art. 76 EPC:
**11742895.3 / 2 533 768**

(71) Applicant: **Ameritox Limited Partnership
Baltimore, MD 21202 (US)**

(72) Inventors:
• **LEIDER, Harry
  Baltimore, MD 21202 (US)**
• **LINDEN, Ariel
  Baltimore, MD 21202 (US)**

(74) Representative: **Shaw, Daniel John
Reddie & Grosse LLP
16 Theobalds Road
London WC1X 8PL (GB)**

Remarks:
This application was filed on 12-06-2015 as a
divisional application to the application mentioned
under INID code 62.

(54) **METHODS OF NORMALISING MEASURED OXYCODONE CONCENTRATIONS AND TESTING FOR NON-COMPLIANCE WITH A TREATMENT REGIMEN**

(57)     Methods for monitoring subject compliance with a prescribed treatment regimen are disclosed. In one embodiment, the method comprises measuring a drug level in fluid of a subject and normalizing said measured drug level as a function of one or more parameters associated with the subject. The normalized drug level is compared to a reference value and associated confidence intervals or to a concentration range. The reference value and associated confidence intervals and/or the concentration range may be normalized based on one or more parameters associated with subjects in a reference population.

Fig. 1A

EP 2 942 055 A1

**Fig. 1B** Normalized Urinary Oxycodone

**Fig. 1C** Raw Urinary Oxycodone, Bonferroni-Adjusted

**Fig. 1D** Normalized Urinary Oxycodone, Bonferroni-Adjusted

**Description**

FIELD OF THE INVENTION

The present invention provides methods for detecting and quantifying a subject's opioid use by, inter alia, testing a biological sample from said subject.

BACKGROUND OF THE INVENTION

Although hydrocodone stands as the most prescribed opioid in the United States, the opioid that is responsible for the most emergency department (ED) visits in the United States is oxycodone. According to the Drug Abuse Warning Network, approximately 77,000 ED visits in 2007 were due to the nonmedical use of oxycodone. The 2007 National Survey on Drug Use and Health estimates that 4.3 million Americans will abuse OXYCONTIN® sometime during the course of their lifetime. Given the propensity for abuse of oxycodone containing medications and high incidence of ED visits associated with abuse, monitoring patients for compliance while being prescribed a pain regimen is an important component of their care.

Because of known dependency risks, subjects on opioid therapy regimens are typically screened periodically to monitor compliance and efficacy of the prescribed therapy. Due to the limits of known screening techniques, however, subjects misusing the prescribed opioid often pass basic screening tests performed at a clinic and continue to receive the opioid. Furthermore, patients treated with opioids for the management of chronic pain also have been documented to under-report their use of medications. As a result, health care professionals often use external sources of information such as interviews with the subject's spouse and/or friends, review of the subject's medical records, input from prescription monitoring programs, and testing of biological samples (e.g., fluids) to detect misuse of drugs and non-compliance with the prescribed opioid regimen.

Known drug screening methods generally can detect the presence or absence of a drug in a sample. Samples of fluids are generally obtained from the subject, for example, urine, blood, or plasma. Such known screening methods do not, however, enable the health care professional reviewing the lab result to determine whether the subject is non-compliant with a prescribed drug regimen.

SUMMARY OF THE INVENTION

In various embodiments, the present invention provides methods for detecting or monitoring a subject's non-compliance with a prescribed drug regimen. In a related embodiment, the drug is an opioid, for example oxycodone, controlled-release oxycodone (OXYCONTIN®), or a metabolite of oxycodone.

In one embodiment, the invention provides a method of normalizing drug levels measured in fluid of a subject or in fluid from members of a population, for example as a function of one or more parameters such as fluid pH, fluid specific gravity, fluid creatinine concentration, height, weight, age, body mass index, gender, lean body mass, and body surface area.

In other embodiments, the invention provides a method of identifying a subject at risk of drug misuse by comparing a drug concentration in fluid of the subject to a reference level, to a reference value and associated confidence interval(s), and/or to a reference concentration range for the drug.

In still other embodiments, the invention provides a method of reducing the risk of drug misuse in a subject by reducing a prescribed daily dose of a drug for the subject or counseling the subject if the drug concentration in fluid of the subject falls outside the confidence intervals or concentration range for the daily dose of the drug.

In other embodiments, the invention provides a method of identifying a risk of drug misuse in a population by comparing normalized drug levels in subjects of the population to a normalized reference drug value and associated confidence intervals or to a normalized concentration range for a daily dose of the drug.

These and other embodiments of the invention will be disclosed in further detail herein below.

BRIEF DESCRIPTION OF THE DRAWINGS

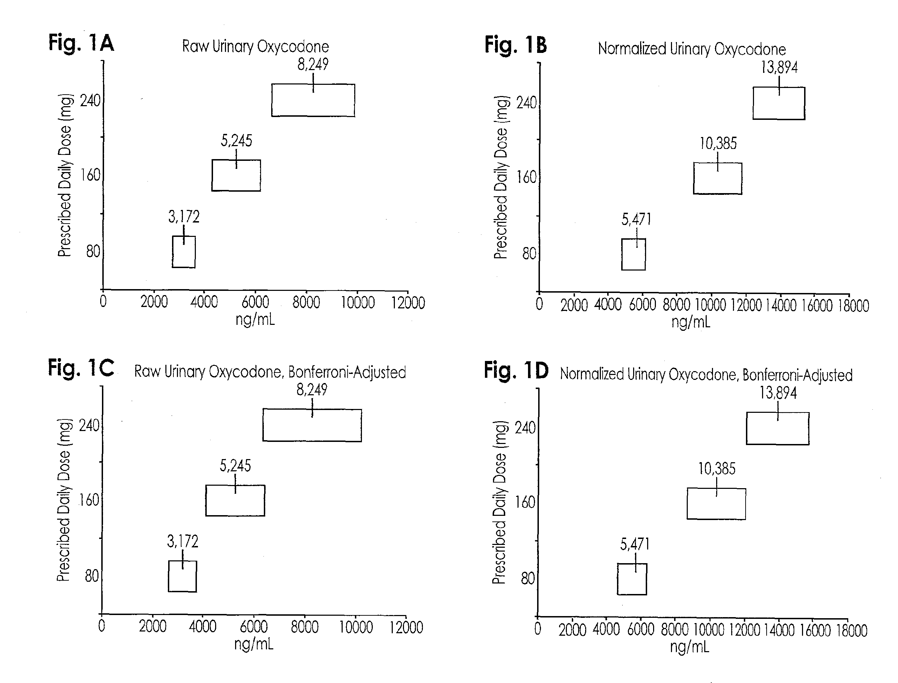

FIG. 1 shows raw (FIGs. 1A, 1C) and normalized (FIGs. 1B, 1D) median reference urine oxycodone estimates and corresponding 95% confidence intervals for each median estimate.

DETAILED DESCRIPTION OF THE INVENTION

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any manner. Embodiments illustrated under any heading may be combined with embodiments illustrated under any other heading.

The use of numerical values in the various quantitative values specified in this application, unless expressly indicated otherwise, are stated as approximations as though the minimum and maximum values within the stated ranges were both preceded by the word "about." Also, the disclosure of ranges is intended as a continuous range including every value between the minimum and maximum values recited as well as any ranges that can be formed by such values. Also disclosed herein are any and all ratios (and ranges of any such ratios) that can be formed by dividing a disclosed numeric value into any other disclosed numeric value. Accordingly, the skilled person will appreciate that many such ratios, ranges, and ranges of ratios can be unambiguously derived from the numerical values presented herein and in all instances such ratios, ranges, and ranges of ratios represent various embodiments of the present invention.

Therapeutic Regimens

In one embodiment, the present invention provides a method of detecting non-compliance with a prescribed opioid regimen in a subject. The term "non-compliance" as used herein refers to any substantial deviation from a course of treatment that has been prescribed by a physician, nurse, nurse practitioner, physician's assistant, or other health care professional. A substantial deviation from a course of treatment may include any intentional or unintentional behavior by the subject that increases or decreases the amount, timing or frequency of opioid ingested compared to the prescribed therapy. Non-limiting examples of substantial deviations from a course of treatment include: taking more of the opioid than prescribed, taking less of the opioid than prescribed, taking the opioid more often than prescribed, taking the opioid less often than prescribed, intentionally diverting at least a portion of the prescribed opioid, unintentionally diverting at least a portion of the prescribed opioid, etc. For example, a subject substantially deviates from a course of treatment by taking about 5% to about 1000% of the prescribed daily dose or prescribed drug regimen, for example about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 105%, about 110%, about 115%, about 120%, about 125%, about 150%, about 175%, about 200%, about 225%, about 250%, about 275%, about 300%, about 350%, about 400%, about 450%, about 500%, about 550%, about 600%, about 650%, about 700%, about 750%, about 800%, about 850%, about 900%, about 950%, or about 1000% of the prescribed drug regimen. A subject may also substantially deviate from a course of treatment by taking about 5% to about 1000% more or less than the prescribed dose, for example about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 125%, about 150%, about 175%, about 200%, about 225%, about 250%, about 275%, about 300%, about 350%, about 400%, about 450%, about 500%, about 550%, about 600%, about 650%, about 700%, about 750%, about 800%, about 850%, about 900%, about 950%, or about 1000% less than the prescribed dose. A subject may also substantially deviate from a course of treatment by, for example, taking the prescribed dose of an opioid about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 125%, about 150%, about 175%, about 200%, about 225%, about 250%, about 275%, about 300%, about 350%, about 400%, about 450%, about 500%, about 550%, about 600%, about 650%, about 700%, about 750%, about 800%, about 850%, about 900%, about 950%, or about 1000% more often or less often than specified in the course of treatment or prescribed in the drug regimen.

In another embodiment, a subject according to the present invention is prescribed a daily dose of a drug. The term "daily dose" or "prescribed daily dose" as used herein refers to any periodic administration of a drug to the subject over a given period of time, for example per hour, per day, per every other day, per week, per month, per year, etc. Preferably the daily dose or prescribed daily dose is the amount of the drug prescribed to a subject in any 24-hour period. The drug may be administered according to any method known in the art including, for example, orally, intravenously, topically, transdermally, subcutaneously, rectally, etc. The prescribed daily dose of the drug may be approved by the Food & Drug Administration ("FDA") for a given indication. In the alternative, a daily dose or a prescribed daily dose may be an unapproved or "off-label" use for a drug for which FDA has approved other indications. As a non-limiting example, FDA has approved oxycodone HCl controlled-release tablets (OXYCONTIN®) for use in the management of moderate to severe pain in 10 mg, 15 mg, 20 mg, 30 mg, 40 mg, 60 mg, 80 mg, 160 mg tablets. Any use of oxycodone HCl controlled-release tablets (OXYCONTIN®) other than to manage moderate to severe pain is an "off-label" use.

In various embodiments, methods according to the present invention involve the step of determining a prescribed dose of a drug. The term "determining a prescribed dose" as used herein refers to any method known to those in the art to ascertain, discover, deduce, or otherwise learn the dose of a particular drug that has been prescribed to the subject. Non-limiting examples include subject interview, consultation with the subject's medical history, consultation with another health care professional familiar with the subject, consultation with a medical record associated with the subject, etc.

The term "drug" as used herein refers to an active pharmaceutical ingredient ("API") and its metabolites, decomposition products, enantiomers, diastereomers, derivatives, etc.

In one embodiment, the drug is an opioid. The term "opioid" as used herein refers to any natural, endogenous, synthetic, or semi-synthetic compound that binds to opioid receptors. Non-limiting examples of opioids include: codeine, morphine, thebaine, oripavine, diacetylmorphine, dihydrocodeine, hydrocodone, hydromorphone, nicomorphone, oxycodone, oxymorphone, fentanyl, alphamethylfentanyl, alfentanil, sufentanil, remifentanil, carfentanyl, ohmefentanyl, pethidine, keobemidone, desmethylprodine, ("MPPP"), allylprodine, prodine, 4-phenyl-1-(2-phenylethyl)piperidin-4-yl acetate ("PEPAP"), propoxyphene, dextropropoxyphene, dextromoramide, bezitramide, piritramide, methadone, dipipanone, levomathadyl acetate ("LAAM"), difenoxin, diphenoxylate, loperamide, dezocine, pentazocine, phenazocine, buprenorphine, dihydroetorphine, etorphine, butorphanol, nalbuphine, levorphanol, levomethorphan, lefetamine, meptazinol, tilidine, tramadol, tapentadol, nalmefene, naloxone, naltrexone, methadone, oxazepam, lorazepam, alprazolam, diazepam, derivatives thereof, metabolites thereof, prodrugs thereof, controlled-release formulations thereof, extended-release formulations thereof, sustained-release formulations thereof, and combinations of the foregoing.

In one embodiment, a method according the present invention confirms a subject's non-adherence to a chronic opioid therapy ("COT"). The term "chronic opioid therapy" as used herein refers to any short-term, mid-term, or long-term treatment regimen comprising at least one opioid. As a non-limiting example, a subject suffering chronic pain may ingest a daily dose of oxycodone to relieve persistent pain resulting from trauma, chronic conditions, etc. COT is generally prescribed to a subject in need of such therapy; subjects on COT are typically monitored periodically by a health care professional for addiction, tolerance, or other common outcomes associated with COT. In one embodiment, a method according to the present invention assists a health care professional in confirming a subject's adherence or non-adherence to a COT regimen.

Subjects on COT sometimes develop an addiction to the prescribed opioid. Studies have shown that a subject on COT is more likely to develop an addiction to a prescribed opioid when he or she has a history of aberrant drug-related behavior, or is at high risk of aberrant drug-related behavior. The term "aberrant drug-related behavior" as used herein refers to any behavioral, genetic, social, or other characteristic of the subject that tends to predispose the subject to development of an addiction for an opioid. Non-limiting examples of such risk factors include a history of drug abuse, a history of opioid abuse, a history of non-opioid drug abuse, a history of alcohol abuse, a history of substance abuse, a history of prescription drug abuse, a low tolerance to pain, a high rate of opioid metabolism, a history of purposeful over-sedation, negative mood changes, intoxicated appearance, an increased frequency of appearing unkempt or impaired, a history of auto or other accidents, frequent early renewals of prescription medications, a history of or attempts to increasing dose without authorization, reports of lost or stolen medications, a history of contemporaneously obtaining prescriptions from more than one doctor, a history of altering the route of administering drugs, a history of using pain relief medications in response to stressful situations, insistence on certain medications, a history of contact with street drug culture, a history of alcohol abuse, a history of illicit drug abuse, a history of hoarding or stockpiling medications, a history of police arrest, instances of abuse or violence, a history of visiting health care professionals without an appointment, a history of consuming medications in excess of the prescribed dose, multiple drug allergies and/or intolerances, frequent office calls and visits, a genetic mutation that up-regulates or down-regulates production of drug metabolizing enzymes, a reduced-function CYP2D6 allele, and/or a non-functional CYP2D6 allele.

In one embodiment, the present invention assists a health care professional in assessing a risk that a subject is misusing a prescribed drug. For example, a method of the present invention comprising measuring a fluid drug concentration, normalizing the fluid drug concentration, and comparing said normalized drug concentration to an expected concentration range or normalized reference value and optionally to upper and lower confidence intervals associated with said normalized reference value to calculate a probability that the subject has misused the prescribed drug. In a related embodiment, a healthcare worker can intervene (e.g. via counseling, modifying the subject's regiment/dose, etc.) in the subject's misuse on the basis of the risk assessment.

Sample Measurement

Methods according to the present invention may be used to determine the amount of a wide variety of drugs in fluids of a subject. When the fluid analyzed is urine, for example, methods according to the present invention may be used to determine the amount of any drug that can be measured in a urine sample.

In one embodiment, the amount of a drug in a subject is determined by analyzing a fluid of the subject. The term "fluid" as used herein refers to any liquid or pseudo-liquid obtained from the subject. Non-limiting examples include urine, blood, plasma, saliva, mucus, and the like. In one embodiment, the fluid is urine.

Determining the amount of a drug in fluid of the subject may be accomplished by use of any method known to those skilled in the art. Non-limiting examples for determining the amount of a drug in fluid of a subject include fluorescence polarization immunoassay ("FPIA," Abbott Diagnostics), mass spectrometry (MS), gas chromatography-mass spectrometry (GC-MS-MS), liquid chromatography-mass spectrometry (LC-MS-MS), and the like. In one embodiment, LC-MS-MS methods known to those skilled in the art are used to determine a raw level, amount, or concentration of a drug in urine of the subject. In one embodiment, a raw level or concentration of a drug in fluid of a subject is measured and reported as a ratio, percent, or in relationship to the amount of fluid. The amount of fluid may be expressed as a unit volume, for example, in L, mL, μL, pL, ounce, etc. In one embodiment, the raw amount of a drug in fluid of a subject may be expressed as an absolute level or value, for example, in g, mg, μg, ng, pg, etc.

In one embodiment, the level, concentration, or amount of a drug determined in fluid of a subject is normalized. The term "normalized" as used herein refers to a level or concentration of a drug that has been adjusted to correct for one or more parameters associated with the subject. Such a parameter may include, for example, a characteristic of the subject, a genetic trait of the subject, a behavioral predisposition of the subject, a measurable or quantifiable property associated with the subject, and the like. For example, a small percentage of the U.S. population feature variations in the cytochrome p450 allele. Broadly, an individual may have a normal CYP2D6 allele, a reduced-function CYP2D6 allele, or a non-functional CYP2D6 allele. As a result, some individuals express more or less (or no) CYP2D6 enzyme, which is an important factor in the metabolism of certain drugs. An individual that expresses more CYP2D6 enzyme is therefore expected to have a lower concentration of certain drugs in fluid measurements compared to individuals with normal levels of CYP2D6 enzymes. Normalizing measurements to account for such variations allows for more accurate comparisons. In one embodiment, one or more parameters associated with the subject is measured in order to normalize the drug concentration or drug level. Non-limiting examples of parameters include: sample fluid pH, sample fluid specific gravity, sample fluid creatinine concentration, subject height, subject weight, subject age, subject body mass index, subject gender, subject lean body mass, and subject body surface area. Parameters may be measured by any means known in the art. For example, sample fluid pH may be measured using a pH meter, litmus paper, test strips, etc.

In one embodiment, the raw drug concentration measured in fluid of the subject is normalized as a function of sample fluid specific gravity according to the following equation:

$$[N] = [C] \times ((SG_N\text{-}1.00)/(SG\text{-}1.00)), \qquad (I)$$

where [N] is the normalized drug level, [C] is the raw drug concentration in ng/mL, SGN is the normalized fluid specific gravity, and SG is the sample fluid specific gravity.

In another embodiment, an estimated plasma concentration is determined from a raw drug concentration level as a function of sample fluid specific gravity, the subject's lean body weight, and the sample fluid's pH according to the following equation:

$$[ETPC] = \{ 0.58 \times ([C] \times ((SG_N\text{-}1.00)/(SG\text{-}1.00)\}/\{(LBW/122) \times (154857 \times (pH\text{-}5.06))\}, \qquad (II)$$

where [ETPC] is the normalized estimated plasma concentration, [C] is the raw drug concentration in ng/mL, SGN is the normalized fluid specific gravity, SG is the specific gravity of the sample fluid, pH is the pH of the sample fluid, and LBW is the subject's lean body weight.

In another embodiment, a normalized drug level is determined from a raw drug concentration as a function of the specific gravity of the sample fluid and the subject's weight according to the following equation:

$$[N] = [C] \times ((SG_N\text{-}1.00)/(SG\text{-}1.00)) \times (W/154), \qquad (III)$$

where [N] is the normalized drug level, [C] is the raw drug concentration in ng/mL, SGN is the normalized fluid specific gravity, SG is the specific gravity of the sample, and W is the weight of the subject in pounds.

In another embodiment, a normalized drug level is determined from a raw drug concentration as a function of the specific gravity of the sample, the subject's lean body weight, and the pH of the sample according to the following equation:

$$[N] = [C] \times ((SG_N\text{-}1.00)/(SG\text{-}1.00)) \times (LBW/122) \times (pH/8.5)^{0.56}, \qquad (IV)$$

where [N] is the normalized drug level, [C] is the raw drug concentration in ng/mL, SGN is the normalized fluid specific gravity, SG is the specific gravity of the sample, LBW is the lean body weight of the subject, and pH is the pH of the sample.

In another embodiment, a normalized drug level is determined from a raw drug concentration as a function of the specific gravity of the sample, the subject's lean body weight, and the pH of the sample according to the following equation:

$$[N] = [C] \; x \; ((SG_N\text{-}1.00)/(SG\text{-}1.00) \; x \; (LBW/122) \; x \; (pH/8.50)^{0.56}), \qquad (V)$$

where [N] is the normalized drug level, [C] is the raw drug concentration in ng/mL, SGN is the normalized fluid specific gravity, SG is the specific gravity of the sample, LBW is the lean body weight of the subject in pounds, and pH is the pH of the sample. In a related embodiment, a normalized oxycodone level is determined from a raw urine oxycodone level as a function of the specific gravity of the urine sample, the subject's lean body weight, and the pH of the urine sample according to Equation (V) where SGN is equal to 1.03. In another related embodiment, a normalized oxycodone level is determined from a raw urine oxycodone level as a function of the specific gravity of the urine sample, the subject's lean body weight, and the pH of the urine sample according to Equation (V), wherein the raw urine oxycodone level is measured by GC-MS-MS or LC-MS-MS and represents the total raw urine concentration of oxycodone, oxymorphone, and noroxycodone.

In another embodiment, raw drug concentrations are normalized as a function of the concentration of creatinine in the sample fluid according to the following equation:

$$[N] = [C] \; x \; ((CR_N\text{-}1.00)/(CR\text{-}1.00), \qquad (VI)$$

where [N] is the normalized drug level, [C] is the raw drug concentration in ng/mL, CRN is the normalized concentration of creatinine in fluid of the same type as the sample fluid obtained from the subject, and CR is the raw concentration of creatinine in the sample obtained from the subject.

"Lean body weight" or "LBW" as used herein refers to a subject's weight excluding the weight of the subject's body fat. Lean body weight may be calculated by any of the following equations:

$$LBW = Body \; Weight - Fat \; Weight \qquad (VII)$$

$$LBW = (Body \; Weight) \; x \; (Body \; Weight \; \%)/100 \qquad (VIII)$$

$$LBW = (Subject \; Body \; Weight) \; x \; [1 - (Body \; Fat \; \%)/100] \qquad (IX)$$

Alternatively, lean body weight or LBW may be estimated by any method known in the art. Non-limiting examples of estimations of LBW include:

$$LBW_{men} = (0.32810 \; x \; (body \; weight_{kg})) + (0.33929 \; x \; (height_{cm})) - 29.5336 \qquad (X)$$

$$LBW_{men} = \frac{2.2 \; x \; (2.447\text{-}(0.09516 \; x \; age_{yrs}) + (0.2728 \; x \; height_{cm}) + (0.1528 \; x \; weight_{lbs})}{0.73} \qquad (XI)$$

$$LBW_{women} = (0.29569 \; x \; (body \; weight_{kg})) + (0.41813 \; x \; (height_{cm})) - 43.2933 \qquad (XII)$$

$$LBW_{women} = \{2.2 \; x \; (\text{-}2.097 + (\text{-}2.097 + (0.2715 \; x \; height_{cm}) + (0.1121 \; x \; weight_{lbs})\}/0.73 \qquad (XIII)$$

In one embodiment, the level or concentration of drug in fluid of the subject is normalized as a function of the subject's body surface area. A non-limiting example of one method to estimate a subject's body surface area in square meters is by the equation:

$$BSA = (height_{cm} \times weight_{kg}/3600)^{0.5} \qquad\qquad (XIV)$$

In one embodiment, the concentration or level of drug in fluid of the subject is a steady state concentration or level. The term "steady state" as used herein refers to an equilibrium level or concentration of a drug obtained at the end of a certain number of administrations (e.g. 1 to about 5). Steady state is achieved when the concentration or level of the drug will remain substantially constant if the dose and the frequency of administrations remain substantially constant.

Normalizing Clinical Data

In one embodiment, the amount of drug measured or determined in fluid of a subject is compared to a reference level of the same drug. The term "reference level" as used herein refers to a standard amount of a drug expected to be present in fluid of a subject that has been administered a given dose of the drug. A reference level may be a steady state level of a drug or may be a single point-in-time level of the drug. Generally, the reference level for a drug in one type of fluid may not be the same as the reference level for the same drug in a different type of fluid. For example, a drug that is quickly excreted through urine may have a higher reference level in urine than in blood or plasma. In contrast, a drug that is slowly excreted may have a higher reference level in blood or plasma than in urine.

A reference level for a given drug may be expressed in various ways. In one embodiment, reference levels useful for methods of the present invention are expressed as a mean, median, average, or weighted average reference value. Optionally, confidence intervals for a given reference value may be established by any suitable statistical methods or models. For example, a raw reference value for oxycodone measured in urine may be 3,172 ng/mL for a daily dose of 80 mg. Confidence intervals provide health care professionals useful data for determining whether a given subject's oxycodone urine level correlates well with the subject's prescribed dose. Thus, 95% confidence levels for the above example of, for example, 2,730-3,613 ng/mL provide the health care professional upper and lower boundaries. The 95% upper and lower confidence intervals represent the range of oxycodone urine levels in which samples from 95% of the population compliant with that prescribed daily dose are expected to fall. Other confidence intervals may be established for a given reference value. Non-limiting examples of confidence intervals include about 99.9%, 99.5%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, and 5%. In one embodiment, reference values and associated confidence intervals are established for a plurality of prescribed daily doses of a drug. In a related embodiment, confidence intervals for each prescribed daily dose do not substantially overlap. In one embodiment, each prescribed daily dose differs from other daily doses by at least 50%, at least 75%, at least 100% or at least 150%.

In another embodiment, confidence intervals for each daily dose of a drug do not overlap. The phrase "do not overlap" as used in the present context means, for example, that the lower confidence interval for one daily dose of a drug does not overlap with the upper confidence interval for a second, smaller daily dose of the same drug.

In one embodiment, reference levels are provided as a concentration range. The term "concentration range" as used herein refers to a continuous range of drug concentrations in fluid of a subject who has been administered a given drug. Concentration ranges are generally unique for a given drug and daily dose of that drug. Concentration ranges may be established by any suitable method known to those skilled in the art, and may be based on a single subject or a population of subjects. For example, concentration ranges may be determined based on a reference value of a drug and associated confidence intervals. As a non-limiting example, and as shown in FIG. 1A, a raw concentration range for a 160 mg prescribed daily dose of oxycodone may be 4,311-6,178 ng/mL, derived from a raw reference value of 5,245 ng/mL, an associated 95% lower confidence interval of 4,311 ng/mL and an associated 95% upper confidence interval of 6,178 ng/mL. Such a concentration range may also be reported as a mean, median, or average value with an error value, for example as 5,245 ± 933.5 ng/mL for a 160 mg daily dose of oxycodone. In one embodiment, concentration ranges are established for each of a plurality of daily doses of a drug. In a related embodiment, concentration ranges for each of a plurality of daily doses of a drug do not substantially overlap.

In another embodiment, the concentration ranges for each prescribed daily dose of a drug do not overlap. For example, the upper-bound of the concentration range of one prescribed daily dose of a drug is less than or equal to the lower-bound of the concentration range of a second, higher prescribed daily dose of the same drug. Preferably in the example described, the upper-bound of the concentration range of one prescribed daily dose of a drug is less than but not equal to the lower-bound of the concentration range of a second, higher prescribed daily dose of the same drug.

In one embodiment of the present invention, reference levels, reference values and associated confidence intervals, and/or concentration ranges are derived from a population of subjects. The term "a population" as used herein refers to any group or selection of subjects for which a reference level, reference value and associated confidence intervals, or concentration range is desired. In a related embodiment, one or a plurality of subjects are assigned to a population. As used herein a "plurality of subjects" refers to two or more subjects, for example about 2 subjects, about 3 subjects, about 4 subjects, about 5 subjects, about 6 subjects, about 7 subjects, about 8 subjects, about 9 subjects, about 10 subjects, about 15 subjects, about 20 subjects, about 25 subjects, about 30 subjects, about 35 subjects, about 40 subjects, about 45 subjects, about 50 subjects, about 55 subjects, about 60 subjects, about 65 subjects, about 70 subjects, about 75 subjects, about 80 subjects, about 85 subjects, about 90 subjects, about 95 subjects, about 100 subjects, about 110 subjects, about 120 subjects, about 130 subjects, about 140 subjects, about 150 subjects, about 160 subjects, about 170 subjects, about 180 subjects, about 190 subjects, about 200 subjects, about 225 subjects, about 250 subjects, about 275 subjects, about 300 subjects, about 325 subjects, about 350 subjects, about 375 subjects, about 400 subjects, about 425 subjects, about 450 subjects, about 475 subjects, about 500 subjects, about 525 subjects, about 550 subjects, about 575 subjects, about 600 subjects, about 625 subjects, about 650 subjects, about 675 subjects, about 700 subjects, about 725 subjects, about 750 subjects, about 775 subjects, about 800 subjects, about 825 subjects, about 850 subjects, about 875 subjects, about 900 subjects, about 925 subjects, about 950 subjects, about 975 subjects, about 1000 subjects, about 1250 subjects, about 1500 subjects, about 1750 subjects, about 2000 subjects, about 2250 subjects, about 2500 subjects, about 2750 subjects, about 3000 subjects, about 3500 subjects, about 4000 subjects, about 4500 subjects, about 5000 subjects, about 5500 subjects, about 6000 subjects, about 6500 subjects, about 7000 subjects, about 7500 subjects, about 8000 subjects, about 8500 subjects, about 9000 subjects, about 9500 subjects, or about 10000 subjects. As used herein with respect to a population, the term "subject" is synonymous with the term "member" and refers to an individual that has been assigned to the population. In one embodiment, subpopulations may be established for a plurality of daily doses of a drug.

In one embodiment, a plurality of subjects of a population are each prescribed the same daily dose of a drug. In another embodiment, a plurality of subjects assigned to one subpopulation are each prescribed a first daily dose of a drug while a plurality of subjects assigned to a second, different subpopulation are each prescribed a second, different daily dose of a drug. In one embodiment, a plurality of subjects assigned to a population or subpopulation are each prescribed a daily dose of a drug for a time sufficient to achieve steady state. The term "time sufficient to achieve steady state" refers to the amount of time required, given the pharmacokinetics of the particular drug and the dose administered to the subject, to establish a substantially constant concentration or level of the drug assuming the dose and the frequency of administrations remain substantially constant. The time sufficient to achieve steady state may be determined from literature or other information corresponding to the drug. For example, labels or package inserts for FDA approved drugs often include information regarding typical times sufficient to achieve steady state plasma concentrations from initial dosing. Other non-limiting means to determine the time sufficient to achieve steady state include experiment, laboratory studies, analogy to similar drugs with similar absorption and excretion characteristics, etc.

Assignment of subjects to a population or subpopulation may be accomplished by any method known to those skilled in the art. For example, subjects may be assigned randomly to one of a plurality of subpopulations. In one embodiment, subjects are screened for one or more parameters before or after being assigned to a population. For example, subjects featuring one or more parameters that may tend to affect fluid levels of a drug may be excluded from a population, may not be assigned to a population, may be assigned to one of a plurality of subpopulations, or may be removed from a population or subpopulation during or after a data collection phase of a study. In one embodiment, subjects with reduced-function CYP2D6 alleles or non-functioning CYP2D6 alleles are excluded from a population because such variants are known to affect the normal opioid metabolic rate. In another embodiment, subjects with reduced-function CYP2D6 alleles are assigned to a first subpopulation, subjects with non-functioning CYP2D6 alleles are assigned to a second subpopulation, and subjects with normal CYP2D6 alleles are assigned to a third subpopulation. Other non-limiting examples of exclusion criteria include: histories of substance abuse; significant disease such as cancer, cardiac disease, autoimmune disease, neurological disease, and the like; recent illness; abnormal findings on physical examination, electrocardiogram, laboratory studies, or drug screens; recent history of prescription drug use, over-the-counter ("OTC") drug use, or herbal drug use; allergies or hypersensitivities to naltrexone, opioids, or similar compounds; recent history of use of alcohol, ingestion of grapefruit, ingestion of grapefruit juice, ingestion of caffeine, or ingestion of xanthene-containing products; and participation in other drug therapy or opioid-related clinical trial or study.

In one embodiment, one or more parameters of a plurality of subjects in a population or subpopulation are measured or ascertained. Non-limiting examples of parameters include fluid pH, fluid specific gravity, fluid creatinine concentration, subject height, subject weight, subject age, subject body mass index, subject gender, and subject lean body mass, and subject body surface area. In one embodiment, parameters include fluid specific gravity, fluid pH, subject body weight, subject gender, and subject height. In another embodiment, parameters include fluid specific gravity, fluid pH, and subject lean body mass. Lean body mass may be calculated or estimated as already discussed, supra.

In one embodiment, drug levels are measured in fluid of a plurality of subjects in a population. Drug levels in a subject's fluid may be measured by any suitable means known to those skilled in the art. Non-limiting examples for determining the amount of a drug in fluid of a subject include fluorescence polarization immunoassay ("FPIA," Abbott Diagnostics), mass spectrometry (MS), gas chromatography-mass spectrometry (GC-MS-MS), liquid chromatography-mass spectrometry (LC-MS-MS), and the like. In one embodiment, LC-MS-MS methods known to those skilled in the art are used to determine the amount of a drug in urine of the subject. In another embodiment, the concentration of a drug in fluid of a subject is measured and reported as a ratio, percent, or in relationship to the amount of fluid. The amount of fluid may be expressed as a unit volume, for example, in L, mL, μL, pL, ounce, etc. In one embodiment, the amount of a drug in fluid of a subject may be expressed as an absolute level or value, for example, in g, mg, μg, ng, pg, etc.

In one embodiment, the raw drug concentrations measured in fluid of a plurality of subjects in a population are normalized as a function of sample fluid specific gravity according to the following equation:

$$[N] = [C] \; x \; ((SG_N\text{-}1.00)/(SG\text{-}1.00)), \qquad\qquad (XV)$$

where [N] is the normalized drug level, [C] is the raw drug concentration in ng/mL, SGN is the normalized fluid specific gravity, and SG is the sample fluid specific gravity.

In another embodiment, an estimated plasma concentration is determined from the raw drug concentration levels in a plurality of subjects in a population as a function of sample fluid specific gravity, the subject's lean body weight, and the sample fluid's pH according to the following equation:

$$[ETPC] = \{ \; 0.58 \; x \; ([C] \; x \; ((SG_N\text{-}1.00)/(SG\text{-}1.00)\}/\{(LBW/122) \; x \; (154857 \; x \; (pH\text{-}5.06))\}, \qquad (XVI)$$

where [ETPC] is the normalized estimated plasma concentration, [C] is the raw drug concentration in ng/mL, SGN is the normalized fluid specific gravity, SG is the specific gravity of the sample fluid, pH is the pH of the sample fluid, and LBW is the subject's lean body weight.

In another embodiment, normalized drug levels for a plurality of subjects in a population are determined from raw drug concentrations as a function of the specific gravity of the sample fluid and the subject's weight according to the following equation:

$$[N] = [C] \; x \; ((SG_N\text{-}1.00)/(SG\text{-}1.00)) \; x \; (W/154), \qquad\qquad (XVII)$$

where [N] is the normalized drug level, [C] is the raw drug concentration in ng/mL, SGN is the normalized fluid specific gravity, SG is the specific gravity of the sample, and W is the weight of the subject in pounds.

In another embodiment, normalized drug levels are determined from the raw drug concentrations of a plurality of subjects in a population as a function of the specific gravity of the sample, the subject's lean body weight, and the pH of the sample according to the following equation:

$$[N] = [C] \; x \; ((SG_N\text{-}1.00)/(SG\text{-}1.00)) \; x \; (LBW/122) \; x \; (pH/8.50)^{0.56}, \qquad (XVIII)$$

where [N] is the normalized drug level, [C] is the raw drug concentration in ng/mL, SGN is the normalized fluid specific gravity, SG is the specific gravity of the sample, LBW is the lean body weight of the subject, and pH is the pH of the sample.

In another embodiment, raw drug concentrations measured in fluid in a plurality of subjects are normalized as a function of the specific gravity of the sample, the subject's lean body weight, and the pH of the sample according to the following equation:

$$[N] = [C] \; x \; ((SG_N\text{-}1.00)/(SG\text{-}1.00) \; x \; (LBW/122) \; x \; (pH/8.50)^{0.56}), \qquad (XIX)$$

where [N] is the normalized drug level, [C] is the raw drug concentration in ng/mL, SGN is the normalized fluid specific gravity, SG is the specific gravity of the sample, LBW is the lean body weight of the subject in pounds, and pH is the pH of the sample. In a related embodiment, raw oxycodone levels measured in urine of a plurality of subjects is normalized as a function of the specific gravity of the urine sample, the subject's lean body weight, and the pH of the urine sample according to Equation (XIX) where SGN is equal to 1.03. In another related embodiment, raw oxycodone levels measured in urine of a plurality of subjects are normalized as a function of the specific gravity of the urine sample, the subject's lean body weight, and the pH of the urine sample according to Equation (XVIII), wherein the raw urine oxycodone level is measured by GC-MS-MS or LC-MS-MS and represents the total raw urine concentration of oxycodone, oxymorphone, and noroxycodone.

In another embodiment, normalized drug levels for a plurality of subjects in a population are determined from raw drug concentrations as a function of the concentration of creatinine in the sample fluid according to the following equation:

$$[N] = [C] \; x \; ((CR_N\text{-}1.00)/(CR\text{-}1.00), \qquad (XX)$$

where [N] is the normalized drug level, [C] is the raw drug concentration in ng/mL measured in fluid from a plurality of subjects, CRN is the normalized concentration of creatinine in fluid of the same type as the sample fluid obtained from the plurality of subjects, and CR is the raw concentration of creatinine in the sample obtained from a plurality of subjects.

"Lean body weight" or "LBW" as used herein refers to a subject's weight or excluding the weight of the subject's body fat. Lean body weight may be calculated by any of the following equations:

$$LBW = Body \; Weight - Fat \; Weight \qquad (XXI)$$

$$LBW = (Body \; Weight) \; x \; (Body \; Weight \; \%)/100 \qquad (XXII)$$

$$LBW = (Subject \; Body \; Weight) \; x \; [1 - (Body \; Fat \; \%)/100] \qquad (XXIII)$$

Alternatively, lean body weight or LBW may be estimated by any method known in the art. Non-limiting examples of estimations of LBW include:

$$LBW_{men} = (0.32810 \; x \; (body \; weight_{kg})) + (0.33929 \; x \; (height_{cm})) - 29.5336 \qquad (XXIV)$$

$$LBW_{men} = \frac{2.2 \; x \; (2.447\text{-}(0.09516 \; x \; age_{yrs}) + (0.2728 \; x \; height_{cm}) + (0.1528 \; x \; weight_{lbs})}{0.73} \qquad (XXV)$$

$$LBW_{women} = (0.29569 \; x \; (body \; weight_{kg})) + (0.41813 \; x \; (height_{cm})) - 43.2933 \qquad (XXVI)$$

$$LBW_{women} = \frac{2.2 \; x \; (\text{-}2.097 + (\text{-}2.097 + (0.2715 \; x \; height_{cm}) + (0.1121 \; x \; weight_{lbs})}{0.73} \qquad (XXVII)$$

In one embodiment, the level or concentration of drug in fluid of a plurality of subjects is normalized as a function of the subject's body surface area. A non-limiting example of one means to estimate a subject's body surface area in square meters is by the equation:

$$BSA = (height_{cm} \; x \; weight_{kg}/3600)^{0.5} \qquad (XXVIII)$$

In one embodiment, the concentration or level of drug in fluid of a plurality of subjects is a steady state concentration or level.

In one embodiment, a drug reference value for a population is determined by aggregating drug concentrations or drug levels for a plurality of subjects assigned to a population. In one embodiment, a normalized drug reference value for a population is determined from aggregation of a plurality of normalized drug concentrations or drug levels of a plurality of subjects assigned to the population. Aggregation of drug concentrations, drug levels, normalized drug concentrations, and/or normalized drug levels may be accomplished by any suitable method known to those skilled in the art. For example, a drug level for a population may be determined as the mean, median, average, or weighted average of a plurality of drug levels measured in a plurality of subjects assigned to the population. In another embodiment, confidence intervals for a reference drug value may be determined for a population. Non-limiting examples of confidence intervals include 99.9%, 99.5%, 99%, 98%, 97%, 96%, 95%, 94%, 93%, 92%, 91%, 90%, 85%, 80%, 75%, 70%, 65%, 60%, 55%, 50%, 45%, 40%, 35%, 30%, 25%, 20%, 15%, 10%, and 5%. In one embodiment, reference values and associated confidence intervals for a population are established for a plurality of daily doses of a drug. In a related embodiment, confidence intervals for each daily dose do not substantially overlap. In one embodiment, confidence intervals for each daily dose of a drug do not overlap.

In one embodiment, reference levels for a population are provided as a normalized concentration range. Normalized concentration ranges for a population may be established by any suitable method known to those skilled in the art. For example, normalized concentration ranges may be determined based on a normalized reference value of a drug for a population and the associated confidence intervals. As a non-limiting example, as shown in FIG. 1B, a normalized concentration range for a population of subjects prescribed a 160 mg daily dose of oxycodone may be 9,004-11,765; derived from a normalized reference value of 10,385 and an associated 95% lower confidence interval of 9004 and an associated 95% upper confidence interval of 11,765. Such a normalized concentration range may also be reported as a mean, median, or average value with an error value, for example as 9,004 ± 1,381 for a prescribed 160 mg daily dose of oxycodone. In one embodiment, normalized concentration ranges for a population are established for each of a plurality of prescribed daily doses of a drug. In a related embodiment, normalized concentration ranges for each of a plurality of prescribed daily doses of a drug do not substantially overlap. In one embodiment, normalized concentration ranges for each of a plurality of prescribed daily doses of a drug do not overlap.

In one embodiment, a normalized concentration range is determined from the aggregation of a plurality of normalized fluid drug levels. For example, a normalized concentration range for a given daily dose of oxycodone is determined as the average of a plurality of oxycodone drug levels of subjects in a population. Standard statistical methods known to those skilled in the art may be used to determine a concentration range, for example, using standard deviations, Bonett-Price analysis, or Bonferroni adjustments for multiple comparisons.

Normalization of concentration ranges can be used to distinguish concentration ranges for a plurality of daily doses of a drug. For example, FIG. 1 shows median urinary concentrations (measured by LC-MS-MS) and associated 95% confidence intervals for three daily doses of controlled-release oxycodone (OXYCONTIN®): 80 mg, 160 mg, and 240 mg. The data in FIG. 1A have not been normalized nor adjusted by Bonferroni adjustments for multiple comparisons. The data in FIG. 1B have been normalized as a function of urine pH, urine specific gravity, and lean body weight and shows greater distinction between the three daily doses. The data in FIG. 1C have not been normalized but has been adjusted by Bonferroni adjustments for multiple comparisons. In this case the Bonett-Price 95% confidence intervals overlap: the upper confidence interval for a prescribed 160 mg daily dose overlaps with the lower confidence interval for 240 mg. When the same data are normalized as a function of urine pH, urine specific gravity, and lean body mass and then adjusted by Bonferroni adjustments for multiple comparisons (FIG. 1D), Bonett-Price 95% confidence intervals for the three daily doses no longer overlap.

In one embodiment, a normalized reference drug level and associated confidence intervals are determined from the aggregation of a plurality of normalized fluid drug levels. For example, a normalized reference drug level for a given daily dose of oxycodone is determined as the average of a plurality of fluid oxycodone levels of subjects in a population. Standard statistical methods may then be used to determine upper and lower confidence intervals, for example, using standard deviations or methods according to Bonett and Price, and optionally Bonferroni adjustments for multiple comparisons.

In one embodiment, reference drug median estimates and associated confidence intervals are established according to the methods of Bonett and Price, for example as described in Psychological Methods, vol. 7, pp. 370-383 (2002), J. Stat. Comput. Simul., vol. 68, pp. 295-305 (2001), and J. Stat. Comput. Simul., vol. 72, pp. 119-124 (2002), each of which are incorporated herein by reference.

Bonferroni adjustments, also sometimes referred to as the Bonferroni method, allow multiple comparisons to be made while maintaining a satisfactory overall confidence coefficient.

Comparison to a Reference Level

In one embodiment, a subject's non-compliance with a prescribed treatment protocol or treatment regimen is confirmed by comparing the subject's fluid drug level to a reference drug level. The method may be used in combination with any other method known to those skilled in the art for detecting a subject's potential non-compliance with a prescribed treatment protocol. Non-limiting examples of such methods include: interviews with the subject, fluid testing for the presence or absence of detectable levels of a drug, observation of the subject's behavior, appreciating reports of diversion of the subject's prescribed drug to others, etc.

In one embodiment, the subject's fluid drug level is normalized for one or more parameters. In another embodiment, the reference drug level is normalized for one or more parameters. In one embodiment, the subject's fluid drug level and the reference drug level are normalized for one or more parameters. In one embodiment, the subject's fluid drug level and the reference drug level are both normalized as a function of the same set of one or more parameters. For example, in one embodiment, the subject's fluid drug level is normalized as a function of fluid pH, fluid specific gravity, and subject body weight; and the reference drug level is normalized as a function of fluid pH, fluid specific gravity, and subject body weight.

In one embodiment, a subject's non-compliance is confirmed if the subject's fluid drug level or concentration falls outside the reference drug level, reference drug value and associated confidence intervals, and/or reference drug concentration range for the daily dose prescribed to the subject. In a related embodiment, the subject's fluid level or concentration is normalized for one or more parameters associated with the subject. In another related embodiment, the reference drug level, reference drug value and associated confidence intervals, and/or reference drug concentration range is normalized for one or more parameters associated with the members of the population. In one embodiment, the subject's fluid drug level or concentration is normalized and the reference drug level, reference drug value and associated confidence intervals, and/or reference drug concentration range is also normalized. In one embodiment, the subject's fluid drug level is a steady state fluid drug level. In another embodiment, the reference drug level, reference drug value and associated confidence intervals, and/or reference drug concentration range is determined from steady state fluid drug levels for a plurality of members of the relevant population.

In one embodiment, all fluid drug levels measured in the subject and the plurality of members of the relevant population are steady state fluid drug levels. In one embodiment, the fluid of the subject is the same type of fluid as the fluid of the members of the relevant population. In one embodiment, the fluid is urine.

In one embodiment, a method according to the present invention is used to identify or determine a subject's non-compliance with a prescribed treatment protocol. In a related embodiment, the method comprises measuring a drug concentration in fluid of the subject; measuring one or more parameters associated with the subject; calculating a normalized drug concentration value for the subject as a function of the drug concentration and the one or more measured parameters; developing, in a population that does not include the subject, a normalized reference drug concentration range corresponding to the prescribed treatment protocol; comparing the normalized drug concentration value of the subject to the normalized reference drug concentration range; and determining that the subject is non-compliant if the normalized drug concentration of the subject falls outside of the normalized reference drug concentration range.

In one embodiment, a method according to the present invention is used to identify a subject at high risk of oxycodone misuse. In a related embodiment, the method comprises determining a prescribed daily dose of oxycodone in the subject; measuring a concentration of oxycodone in urine of the subject; measuring one or more parameters in the subject and/or the urine of the subject; calculating a normalized oxycodone concentration value as a function of the oxycodone concentration and the one or more parameter; comparing the normalized oxycodone concentration value to a normalized mean or median estimate corresponding to the prescribed daily dose of oxycodone; and identifying the subject at high risk of oxycodone misuse if the normalized oxycodone concentration value falls outside upper and lower confidence levels corresponding to the prescribed daily dose of oxycodone.

In a related embodiment, the normalized mean or median estimate and associated upper and lower confidence levels corresponding to the prescribed daily dose of oxycodone are determined by administering to each of a plurality of members of a population the prescribed daily dose of oxycodone until steady state is achieved; measuring an oxycodone concentration in urine of each member; measuring one or more parameters in each member; normalizing the oxycodone concentration for each member as a function of the one or more parameters; determining the normalized mean or median estimate corresponding to the prescribed daily dose of oxycodone from the normalized oxycodone concentrations of the population; and determining upper and lower confidence intervals for the normalized mean or median estimate.

In one embodiment, a method according to the present invention is used to reduce risk of drug misuse in a subject. In a related embodiment, the method comprises determining a prescribed daily dose of a drug in the subject; measuring a concentration of the drug in urine of the subject; measuring one or more parameters associated with the subject; calculating a normalized drug concentration value as a function of the drug concentration and the one or more parameters; comparing the normalized drug concentration value to a normalized mean or median estimate corresponding to the prescribed daily dose of the drug; identifying the subject at high risk of drug misuse if the normalized drug concentration value falls outside upper and lower confidence levels corresponding to the prescribed daily dose of the drug; and thereafter reducing the prescribed daily dose of the drug in the subject. In a related embodiment, the drug comprises an opioid, for example, oxycodone, controlled-release oxycodone, a metabolite of oxycodone, or combinations thereof.

In one embodiment, a method according to the present invention is used to confirm a subject's non-adherence to a chronic opioid therapy (COT) regimen. In a related embodiment, the method comprises prescribing to the subject a chronic opioid therapy regimen, said regimen comprising a daily dose of an opioid; determining, after a time sufficient to achieve steady state, a normalized opioid concentration value in the subject; developing, in a secondary population that does not include the subject, a normalized mean or median opioid estimate and associated upper and lower confidence intervals corresponding to the prescribed daily dose of the opioid; comparing the normalized opioid concentration value in the subject to the normalized mean or median opioid estimate corresponding to the prescribed daily dose of the opioid; and determining the subject's non-adherence to the chronic opioid therapy regimen if the normalized opioid concentration value in the subject falls outside the confidence intervals corresponding to the prescribed daily dose of the opioid. In a related embodiment, the opioid comprises oxycodone, controlled-release oxycodone, a metabolite of oxycodone, or combinations thereof. In a related embodiment, the subject has a history of or is at high risk of developing aberrant drug-related behavior.

In a related embodiment, the normalized opioid concentration in the subject is determined by measuring a steady state opioid concentration in urine of the subject; measuring one or more parameters in the subject and/or urine of the subject; and calculating the normalized opioid concentration in the subject as a function of the one or more parameters measured in the subject and/or urine of the subject. In a related embodiment, the one or more parameters are selected from the group consisting of urine pH, urine specific gravity, urine creatinine concentration, subject height, subject weight, subject age, subject body mass index, subject gender, subject lean body mass, and subject body surface area.

In one embodiment, a method according to the present invention is used to identify a risk of drug misuse in a population. In a related embodiment, the method comprises assigning a plurality of subjects to a first population; administering to the plurality of subjects a daily dose of a drug; determining a level of said drug in fluid of the plurality of subjects; measuring one or more parameters associated with the plurality of subjects; normalizing said levels of said drug as a function of at least one of said parameters; developing, in a second population that does not include said plurality of subjects, a normalized reference drug level and associated confidence intervals corresponding to the daily dose of the drug; comparing the normalized levels of said drug in the first population to the normalized reference drug level and associated confidence intervals; and identifying a risk of drug misuse in a first population if the normalized levels of said drug in the first population fall outside the associated confidence intervals corresponding to the daily dose of the drug.

In one embodiment, a method according to the present invention provides a probability that a subject is non-compliant with a prescribed drug regimen. In a related embodiment, a raw drug level measured in fluid obtained from a subject is normalized as a function of one or more parameters associated with the subject. A probability that the subject is non-compliant with a prescribed drug regimen is then determined by comparing the normalized drug level to at least one of: a normalized reference drug level associated with the prescribed drug regimen, upper and lower confidence intervals associated with said normalized reference drug level, and/or a normalized concentration range associated with the prescribed drug regimen.

In one embodiment, a refined probability that the subject is non-compliant with a prescribed drug regimen results from the combination of the probability that the subject is non-compliant (e.g., determined by comparing a normalized drug level in fluid of the subject to at least one of a normalized reference drug level associated with the prescribed drug regimen, upper and lower confidence intervals associated with said normalized reference drug level, and/or a normalized concentration range associated with the prescribed drug regimen) with a pretest probability. The phrase "pretest" as used herein refers to any assessment tool known to those skilled in the art other than diagnostic testing of fluid obtained from a subject as set forth herein that tends to predict or demonstrate a subject's compliance with a prescribed drug regimen. In a related embodiment, the pre-test probability is derived from at least one of: administration of one or more questionnaires, administration of one or more standard risk screening instruments, appreciation of one or more aberrant drug behaviors, conducting of one or more interviews with friends of the subject, conducting of one or more interviews with relatives of the subject, conducting of one or more interviews with acquaintances of the subject, and the like. In a related embodiment, the refined probability that a subject is non-compliant results from the multiplication of the pretest probability with the probability determined from comparison of a normalized drug level in fluid of the subject to at least one of a normalized reference drug level associated with the prescribed drug regimen, upper and lower confidence intervals associated with said normalized reference drug level, and/or a normalized concentration range associated with the prescribed drug regimen.

**EXAMPLES**

The following examples are for illustrative purposes only and are not to be construed as limiting the scope of the invention in any respect whatsoever.

EXAMPLE 1

Thirty-six healthy adult, non-smoking subjects, 18-50 years of age, with body mass index between 18-50 years of age with body mass index between 18 and 32 kg/m3 were studied in a single-group, multiple dose study of 80, 160, and 240 mg daily dose controlled-release oxycodone (OXYCONTIN®). Fifteen of the subjects were female; 21 were male. Women were required to have a negative urine pregnancy test before the study initiation as well as to use a medically accepted method of contraception throughout the duration of the study. All participants were screened for phenotypic variation of the CYP2D6 enzymes using a commercially available screening test (PGXL Laboratories, Louisville, KY), and ultrarapid, rapid, and poor metabolizers were excluded from the study. Other exclusions included individuals with histories of substance abuse, significant disease, recent illness, or abnormal finding on physical examination, electrocardiogram, laboratory studies, or drug screens. Additionally, those with recent histories of prescription, over the counter, and herbal drug use were excluded. The subjects with allergies or hypersensitivities to naltrexone, oxycodone, other opioids, or similar compounds were ineligible to participate. These subjects were forbidden to use alcohol, ingest grapefruit, grapefruit juice, caffeine, or xanthene-containing products 48 hours before dosing and during the dosing periods.

Each subject received naltrexone blockade throughout the study (50 mg daily naltrexone dosing was initiated 12 hours before oxycodone administration and continued through day 4) according to the study protocol. The subjects ranged in age from 18 to 50 years (mean = 23.58) and averaged 68 inches in height and 159 pounds in weight. Thirty-two participants were Caucasian.

The subjects were randomized to one of the three dosages of controlled-release oxycodone: 80, 160, or 240 mg/d dosed every 12 hours through day 4. On day 2, two presteady-state urine samples were collected every 12 hours. The half-life of controlled-release oxycodone is 4.5 hours, and most patients will reach steady state after 4-5 half lives of a drug, leaving most patients taking controlled-release oxycodone at steady state before day 3. Previous pharmacokinetic studies have confirmed that upon repeated dosing of controlled-release oxycodone, patients achieve steady-state levels within 24-36 hours. Beginning on day 3 at midnight, urine samples of all subjects were collected through 23:59 on day 4, while subjects were at steady state. A total of 373 urine samples were collected while the subjects were in steady state for each dose of controlled-release oxycodone. In addition, pK blood and oral fluid samples were collected three times on days 3 and 4. Laboratory, medication, physical examination, and adverse event findings were collected in the event of early termination or at the follow-up visit. Raw oxycodone levels in the urine samples were determined by LC-MS-MS and included the total detectable measurements for noroxycodone, oxymorphone, and oxycodone in the urine.

An analysis of medians was conducted for each of the daily dosage groupings using Bonett-Price confidence intervals for both raw and normalized drug levels. An analysis of medians, rather than means in this instance, was more appropriate because (a) the distribution of values in this relatively small sample appears skewed at each dose level; (b) no information as to the true population distribution of drug levels was available, and (c) the analysis of medians is robust to almost any type of non-normality that would likely be encountered in practice. The Bonett-Price confidence interval method was chosen in particular because of its superior performance in simulation experiments of small samples. For comparison purposes, and to establish even more conservative estimates, a Bonferroni adjustment was applied to the confidence intervals.

TABLE 1.

| A | B | C | D | E | F |
|---|---|---|---|---|---|
| Prescribed Daily Dose (mg) | Median Urinary Drug Level | 95% Lower Confidence Interval | 95% Upper Confidence Interval | Bonferroni-Adjusted | |
| | | | | 95% Lower Confidence Interval | 95% Upper Confidence Interval |
| Raw Data (ng/mL) | | | | | |
| 80 | 3,172 | 2,730 | 3,613 | 2,632 | 3,711 |
| 160 | 5,245 | 4,311 | 6,178 | 4,105 | 6,384 |
| 240 | 8,249 | 6,647 | 9,851 | 6,292 | 10,206 |
| Normalized Data | | | | | |
| 80 | 5,471 | 4,796 | 6,147 | 4,646 | 6,296 |
| 160 | 10,385 | 9,004 | 11,765 | 8,699 | 12,071 |
| 240 | 13,894 | 12,426 | 15,361 | 12,101 | 15,686 |

TABLE 1 and corresponding FIGs. 1A-1D represent the results of the study of Example 1. Reference fluid drug levels were calculated as medians from LC-MS-MS measurements of oxycodone levels in each subject assigned to each daily dose subpopulation (column B). Raw (i.e., not normalized) medians for 80, 160, and 240 mg daily doses were 3,172; 5,245; and 8,249 ng/mL, respectively. For each of these median levels, Bonett-Price 95% confidence intervals were calculated (columns C-D). For comparison, Bonferroni-adjusted Bonett-Price 95% confidence intervals were also calculated (columns E-F). A graphical representation of these data is shown in FIGs. 1A and 1C.

Normalized median fluid drug levels were also determined as a function of urine pH, urine specific gravity, and lean body mass (column B). Normalized median urinary oxycodone levels for 80, 160, and 240 mg daily doses of controlled-release oxycodone were 5,471; 10,385; and 13,894, respectively. Bonett-Price 95% confidence intervals (columns C-D) and Bonferroni-adjusted Bonett-Price 95% confidence intervals (columns E-F) were also determined for the normalized median urinary oxycodone levels as shown in TABLE 1. A graphical representation of these data is shown in FIGs. 1B and 1D.

Aspects of the invention are defined in the numbered paragraphs below:

1. A method of identifying a subject as high risk of oxycodone misuse comprising: determining a prescribed daily dose of oxycodone in the subject; measuring a concentration of oxycodone in urine of the subject; measuring one or more parameters in the subject and/or the urine of the subject; calculating a normalized oxycodone value as a function of the oxycodone concentration and the one or more parameter; comparing the normalized oxycodone value to a normalized confidence interval corresponding to the prescribed daily dose of oxycodone; and identifying the subject as high risk of oxycodone misuse if the normalized oxycodone value falls outside an upper and a lower limit of the confidence levels corresponding to the prescribed daily dose of oxycodone.

2. The method of Paragraph 1 wherein the one or more parameter is selected from the group consisting of urine pH, urine specific gravity, urine creatinine concentration, subject height, subject weight, subject age, subject body mass index, subject gender, subject lean body mass, and subject body surface area.

3. The method of Paragraph 1 wherein the concentration of oxycodone is measured using LC-MS-MS or GC-MS-MS.

4. The method of Paragraph 1 wherein the normalized median estimate and associated upper and lower confidence levels corresponding to the prescribed daily dose of oxycodone are determined by: administering to each of a plurality of members of a population the prescribed daily dose of oxycodone until steady state is achieved; measuring an oxycodone concentration in urine of each member; measuring one or more parameters in each member; normalizing the oxycodone concentration for each member as a function of the one or more parameters; determining the normalized median estimate corresponding to the prescribed daily dose of oxycodone from the normalized oxycodone concentrations of the population; and determining upper and lower confidence intervals for the normalized median estimate.

5. The method of Paragraph 4 wherein the plurality of members are assigned to the population based on the presence or absence of one or more exclusion criteria.

6. The method of Paragraph 5 wherein the one or more exclusion criteria are selected from the list comprising CYP2D6 allele variation, histories of substance abuse; significant disease; recent illness; abnormal findings on physical examination, electrocardiogram, laboratory studies, or drug screens; recent history of prescription drug use, over-the-counter drug use, or herbal drug use; allergies or hypersensitivities to naltrexone, opioids, or similar compounds; recent history of use of alcohol, ingestion of grapefruit, ingestion of grapefruit juice, ingestion of caffeine, or ingestion of xanthene- containing products; and participation in another drug therapy or opioid-related clinical trial or study.

7. The method of Paragraph 4 wherein the members do not have a non-functional CYP2D6 allele.

8. The method of Paragraph 4 wherein the members do not have a reduced- function CYP2D6 allele.

9. The method of Paragraph 4 wherein the members do not have a non-functional or a reduced-function CYP2D6 allele.

10. The method of Paragraph 4 wherein the members all of the subjects have a functional CYP2D6 allele.

11. The method of Paragraph 4 wherein the oxycodone concentration is measured using LC-MS-MS or GC-MS-MS.

12. A method of reducing a risk of drug misuse in a subject comprising: determining a prescribed daily dose of a drug in the subject; measuring a concentration of the drug in urine of the subject; measuring one or more parameters associated with the subject; calculating a normalized value as a function of the drug concentration and the one or more parameters; comparing the normalized value to a normalized mean or median estimate corresponding to the prescribed daily dose of the drug; identifying the subject at high risk of drug misuse if the normalized drug concentration falls outside the normalized mean or median estimate corresponding to the prescribed daily dose of the drug; and thereafter altering the daily dose of the drug prescribed to the subject.

13. The method of Paragraph 12 wherein the concentration of the drug is measured using LC-MS-MS or GC-MS-MS.

14. The method of Paragraph 12 wherein the one or more parameters is selected from the group consisting of urine pH, urine specific gravity, urine creatinine concentration, subject height, subject weight, subject age, subject body mass index, subject gender, subject lean body mass, and subject body surface area.

15. The method of Paragraph 12 wherein the drug is an opioid.

16. The method of Paragraph 12 wherein the drug comprises oxycodone.

17. The method of Paragraph 12 wherein the drug comprises controlled-release oxycodone.

18. The method of Paragraph 12 wherein the drug comprises a metabolite of oxycodone.

19. The method of Paragraph 12 wherein the normalized median estimate and associated upper and lower confidence levels corresponding to the prescribed daily dose of the drug are determined by: administering to each of a plurality of members of a population the prescribed daily dose of the drug until steady state is achieved; measuring a drug concentration in urine of each member; measuring one or more parameters in each member; normalizing the drug concentration for each member as a function of the one or more parameters; determining the normalized median estimate corresponding to the prescribed daily dose of the drug from the normalized drug concentrations of the population; and determining upper and lower confidence intervals for the normalized median estimate.

20. The method of Paragraph 19 wherein the drug concentration is measured using LC-MS-MS or GC-MS-MS.

21. The method of Paragraph 19 wherein the drug is an opioid.

22. The method of Paragraph 19 wherein the drug comprises oxycodone.

23. The method of Paragraph 19 wherein the drug comprises controlled-release oxycodone.

24. The method of Paragraph 19 wherein the drug comprises a metabolite of oxycodone.

25. The method of Paragraph 19 wherein the plurality of members are assigned to the population based on the presence or absence of one or more exclusion criteria.

26. The method of Paragraph 25 wherein the one or more exclusion criteria are selected from the list comprising CYP2D6 allele variation, histories of substance abuse; significant disease; recent illness; abnormal findings on physical examination, electrocardiogram, laboratory studies, or drug screens; recent history of prescription drug use, over-the-counter drug use, or herbal drug use; allergies or hypersensitivities to naltrexone, opioids, or similar compounds; recent history of use of alcohol, ingestion of grapefruit, ingestion of grapefruit juice, ingestion of caffeine, or ingestion of xanthene- containing products; and participation in another drug therapy or opioid-related clinical trial or study.

27. The method of Paragraph 19 wherein the members do not have a non- functional CYP2D6 allele.

28. The method of Paragraph 19 wherein the members do not have a reduced- function CYP2D6 allele.

29. The method of Paragraph 19 wherein the members do not have a non- functional or a reduced-function CYP2D6 allele.

30. The method of Paragraph 19 wherein the members all of the subjects have a functional CYP2D6 allele.

31. A method of identifying a risk of drug misuse m a first population comprising: assigning a plurality of subjects to a first population; administering to the plurality of subjects a daily dose of a drug; determining a level of said drug in fluid of the plurality of subjects; measuring one or more parameters associated with the plurality of subjects; normalizing said levels of said drug as a function of at least one of said parameters; developing, in a second population that does not include said plurality of subjects, a normalized reference drug level and associated confidence intervals corresponding to the daily dose of the drug; comparing the normalized levels of said drug in the first population to the normalized reference drug level and associated confidence intervals; and identifying a risk of drug misuse in a first population if the normalized levels of said drug in the first population fall outside the associated confidence intervals corresponding to the daily dose of the drug.

32. The method of Paragraph 31 wherein the second population comprises a plurality of members.

33. The method of Paragraph 32 wherein a value of at least one of the measured parameters associated with the plurality of subjects assigned to the first population differs from a value of at least one of the measured parameters associated with the plurality of members comprising the second population.

34. The method of Paragraph 33 wherein the one or more parameter is selected from fluid pH, fluid specific gravity, fluid creatinine concentration, subject height, subject weight, age, body mass index, gender, lean body mass, and body surface area.

35. The method of Paragraph 31 wherein the plurality of subjects has a high risk of aberrant drug-related behavior.

36. The method of Paragraph 31 wherein the plurality of subjects has a history of aberrant drug-related behavior.

37. The method of Paragraph 31 wherein the drug comprises an opioid.

38. The method of Paragraph 31 wherein the drug comprises oxycodone.

39. The method of Paragraph 31 wherein the drug comprises controlled-release oxycodone.

40. The method of Paragraph 31 wherein the drug comprises a metabolite of oxycodone.

41. The method of Paragraph 31 wherein the level of said drug is determined using LC-MS-MS or GC-MS-MS.

## Claims

1. A method of identifying a subject as high risk of oxycodone misuse comprising:

   determining a prescribed daily dose of oxycodone in the subject;
   measuring a concentration of oxycodone in urine of the subject;
   measuring one or more parameters in the subject and/or the urine of the subject;
   calculating a normalized oxycodone value as a function of the oxycodone concentration and the one or more parameter;
   comparing the normalized oxycodone value to a normalized confidence interval corresponding to the prescribed daily dose of oxycodone; and
   identifying the subject as high risk of oxycodone misuse if the normalized oxycodone value falls outside an upper and a lower limit of the confidence levels corresponding to the prescribed daily dose of oxycodone.

2. The method of Claim 1 wherein the normalized median estimate and associated upper and lower confidence levels corresponding to the prescribed daily dose of oxycodone are determined by:

   measuring an oxycodone concentration in urine of each member of a plurality of members of a population who have been administered the prescribed daily dose of oxycodone until steady state is achieved;
   measuring one or more parameters in each member;
   normalizing the oxycodone concentration for each member as a function of the one or more parameters;
   determining the normalized median estimate corresponding to the prescribed daily dose of oxycodone from the normalized oxycodone concentrations of the population; and
   determining upper and lower confidence intervals for the normalized median estimate.

**3.** A method of reducing a risk of drug misuse in a subject comprising:

determining a prescribed daily dose of a drug in the subject;
measuring a concentration of the drug in urine of the subject;
measuring one or more parameters associated with the subject;
calculating a normalized value as a function of the drug concentration and the one or more parameters;
comparing the normalized value to a normalized mean or median estimate corresponding to the prescribed daily dose of the drug;
identifying the subject at high risk of drug misuse if the normalized drug concentration falls outside the normalized mean or median estimate corresponding to the prescribed daily dose of the drug; and
thereafter altering the daily dose of the drug prescribed to the subject.

**4.** The method of any preceding Claim wherein the one or more parameters is selected from the group consisting of urine pH, urine specific gravity, urine creatinine concentration, subject height, subject weight, subject age, subject body mass index, subject gender, subject lean body mass, and subject body surface area.

**5.** The method of Claim 3 wherein the normalized median estimate and associated upper and lower confidence levels corresponding to the prescribed daily dose of the drug are determined by:

measuring a drug concentration in urine of each member of a plurality of members of a population who have been administered the prescribed daily dose of the drug until steady state is achieved;
measuring one or more parameters in each member;
normalizing the drug concentration for each member as a function of the one or more parameters;
determining the normalized median estimate corresponding to the prescribed daily dose of the drug from the normalized drug concentrations of the population; and
determining upper and lower confidence intervals for the normalized median estimate.

**6.** The method of Claim 2 or Claim 5 wherein the plurality of members are assigned to the population based on the presence or absence of one or more exclusion criteria.

**7.** The method of Claim 6 wherein the one or more exclusion criteria are selected from the list comprising CYP2D6 allele variation, histories of substance abuse; significant disease; recent illness; abnormal findings on physical examination, electrocardiogram, laboratory studies, or drug screens; recent history of prescription drug use, over-the-counter drug use, or herbal drug use; allergies or hypersensitivities to naltrexone, opioids, or similar compounds; recent history of use of alcohol, ingestion of grapefruit, ingestion of grapefruit juice, ingestion of caffeine, or ingestion of xanthene- containing products; and participation in another drug therapy or opioid- related clinical trial or study.

**8.** The method of Claim 2 or Claim 5 wherein the members:

(a) do not have a nonfunctional CYP2D6 allele;
(b) do not have a reduced- function CYP2D6 allele;
(c) do not have a nonfunctional or a reduced-function CYP2D6 allele; or
(d) all of the subjects have a functional CYP2D6 allele.

**9.** A method of identifying a risk of drug misuse in a first population comprising:

assigning a plurality of subjects to a first population, wherein said plurality of subjects have been administered a daily dose of a drug;
determining a level of said drug in fluid of the plurality of subjects;
measuring one or more parameters associated with the plurality of subjects;
normalizing said levels of said drug as a function of at least one of said parameters;
developing, in a second population that does not include said plurality of subjects, a normalized reference drug level and associated confidence intervals corresponding to the daily dose of the drug;
comparing the normalized levels of said drug in the first population to the normalized reference drug level and associated confidence intervals; and
identifying a risk of drug misuse in a first population if the normalized levels of said drug in the first population fall outside the associated confidence intervals corresponding to the daily dose of the drug.

**10.** The method of Claim 9 wherein the second population comprises a plurality of members.

**11.** The method of Claim 10 wherein a value of at least one of the measured parameters associated with the plurality of subjects assigned to the first population differs from a value of at least one of the measured parameters associated with the plurality of members comprising the second population.

**12.** The method of Claim 11 wherein the one or more parameter is selected from fluid pH, fluid specific gravity, fluid creatinine concentration, subject height, subject weight, age, body mass index, gender, lean body mass, and body surface area.

**13.** The method of Claim 9 wherein the plurality of subjects has:

(a) a high risk of aberrant drug-related behavior; or
(b) a history of aberrant drug-related behavior.

**14.** The method of any preceding Claim wherein the drug comprises:

(a) an opioid;
(b) oxycodone;
(c) controlled-release oxycodone; or
(d) a metabolite of oxycodone.

**15.** The method of any preceding Claim wherein the level of said drug is determined using LC-MS-MS or GC-MS-MS.

**Fig. 1A**

Raw Urinary Oxycodone

Prescribed Daily Dose (mg): 240, 160, 80

ng/mL: 0, 2000, 4000, 6000, 8000, 10000, 12000

3,172 5,245 8,249

**Fig. 1B**

Normalized Urinary Oxycodone

Prescribed Daily Dose (mg): 240, 160, 80

ng/mL: 0, 2000, 4000, 6000, 8000, 10000, 12000, 14000, 16000, 18000

5,471 10,385 13,894

**Fig. 1C**

Raw Urinary Oxycodone, Bonferroni-Adjusted

Prescribed Daily Dose (mg): 240, 160, 80

ng/mL: 0, 2000, 4000, 6000, 8000, 10000, 12000

3,172 5,245 8,249

**Fig. 1D**

Normalized Urinary Oxycodone, Bonferroni-Adjusted

Prescribed Daily Dose (mg): 240, 160, 80

ng/mL: 0, 2000, 4000, 6000, 8000, 10000, 12000, 14000, 16000, 18000

5,471 10,385 13,894

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/291468 A1 (LARSON MICHAEL E M [US] ET AL) 26 November 2009 (2009-11-26) | 1-15 | INV. A61K9/20 G01N33/94 |
| Y | * the whole document * * examples 2,3 * * paragraphs [0012], [0017], [0032], [0048], [0062] * | 2,5 | |
| X | M. E.M. LARSON ET AL: "Quantification of a Methadone Metabolite (EDDP) in Urine: Assessment of Compliance", CLINICAL MEDICINE & RESEARCH, vol. 7, no. 4, 1 December 2009 (2009-12-01), pages 134-141, XP055065087, ISSN: 1539-4182, DOI: 10.3121/cmr.2009.859 | 1-15 | |
| Y | * the whole document * * page 139, column 1 - page 140, column 1 * | 2,5 | |
| X | COUTO JOSEPH E ET AL: "Use of an algorithm applied to urine drug screening to assess adherence to an oxycontin regimen", JOURNAL OF OPIOID MANAGEMENT,, vol. 5, no. 6, 1 November 2009 (2009-11-01), pages 359-364, XP009132311, * the whole document * * page 361 * * page 362, column 1, paragraph 3 * * page 363, column 1, paragraph 3 * | 1-15 | TECHNICAL FIELDS SEARCHED (IPC) G01N |
| X | US 5 908 788 A (KELL MICHAEL [US]) 1 June 1999 (1999-06-01) | 1-15 | |
| Y | * the whole document * * column 3, lines 21-25 * * column 17 - column 21 * * column 24, lines 32-60 * | 2,5 | |

-/--

The present search report has been drawn up for all claims

| Place of search | Date of completion of the search | Examiner |
|---|---|---|
| The Hague | 27 July 2015 | Jenkins, Gareth |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 15 17 1980

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE MEDLINE [Online] US NATIONAL LIBRARY OF MEDICINE (NLM), BETHESDA, MD, US; September 2002 (2002-09), BONETT DOUGLAS G ET AL: "Statistical inference for a linear function of medians: confidence intervals, hypothesis testing, and sample size requirements.", XP002742717, Database accession no. NLM12243307 * abstract * & BONETT DOUGLAS G ET AL: "Statistical inference for a linear function of medians: confidence intervals, hypothesis testing, and sample size requirements.", PSYCHOLOGICAL METHODS SEP 2002, vol. 7, no. 3, September 2002 (2002-09), pages 370-383, ISSN: 1082-989X ----- | 2,5 | |

TECHNICAL FIELDS
SEARCHED (IPC)

The present search report has been drawn up for all claims

| Place of search | Date of completion of the search | Examiner |
|---|---|---|
| The Hague | 27 July 2015 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 15 17 1980

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-2015

| Patent document cited in search report | | Publication date | Patent family member(s) | | Publication date |
|---|---|---|---|---|---|
| US 2009291468 | A1 | 26-11-2009 | US | 2005048666 A1 | 03-03-2005 |
| | | | US | 2009291468 A1 | 26-11-2009 |
| | | | US | 2011086428 A1 | 14-04-2011 |
| | | | US | 2015017733 A1 | 15-01-2015 |
| US 5908788 | A | 01-06-1999 | AT | 245814 T | 15-08-2003 |
| | | | AU | 698403 B2 | 29-10-1998 |
| | | | AU | 8087394 A | 23-05-1995 |
| | | | CA | 2175584 A1 | 11-05-1995 |
| | | | DE | 69432976 D1 | 28-08-2003 |
| | | | EP | 0748444 A1 | 18-12-1996 |
| | | | JP | 4389005 B2 | 24-12-2009 |
| | | | JP | H09508967 A | 09-09-1997 |
| | | | US | 5652146 A | 29-07-1997 |
| | | | US | 5908788 A | 01-06-1999 |
| | | | WO | 9512812 A1 | 11-05-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Psychological Methods,* 2002, vol. 7, 370-383
- *J. Stat. Comput. Simul.,* 2001, vol. 68, 295-305
- *J. Stat. Comput. Simul.,* 2002, vol. 72, 119-124